# EUROPEAN PATENT APPLICATION

(11) **EP 3 031 462 A1**
(43) Date of publication of application: **15.06.2016**
(21) Application number: 14834780.0
(22) Date of filing: 05.08.2014
(51) Int. Cl.: A61K 31/765, A61K 31/513, A61K 31/7068, A61K 45/00, A61P 35/00, A61P 43/00

(54) **MEDICINE FOR PREVENTING OR SUPPRESSING SURVIVAL OF CANCER CELLS AND HAVING ORGANIC ACID POLYMER AS ACTIVE INGREDIENT**

(30) Priority: 06.08.2013 JP 2013162872
(71) Applicant: Kyushu University, National University Corporation, Fukuoka-shi, Fukuoka 812-8581 (JP); Sanwa Kagaku Kenkyusho Co., Ltd, Nagoya-shi Aichi 461-8631 (JP)
(72) Inventor: NAKAYAMA, Keiichi, Fukuoka-shi Fukuoka 812-8581 (JP); YUMIMOTO, Kanae, Fukuoka-shi Fukuoka 812-8581 (JP); JOMORI, Takahito, Nagoya-shi Aichi 461-8631 (JP)
(74) Representative: Schulz Junghans Patentanwälte PartGmbB
(86) International application number: PCT/JP2014/070598
(87) International publication number: WO 2015/020040

(57) **Abstract**

The present invention addresses the problem of providing a medicine for preventing or suppressing the survival of cancer cells in a destination tissue. The purpose of the present invention is to provide a medicine for preventing or suppressing the survival of cancer cells in lynphogenous or hematogenous destination tissue, and having an organic acid polymer represented by general formula (I) [(O_{1/2})₃Ge-A-COOH]n (I) (in the formula, n represents an integer of 100-1000, and A represents a lower alkylene group) as an active ingredient thereof. It is preferable for A in general formula (I) of the organic acid polymer to be a C1-3 lower alkylene group, and for the degree of polymerization (ii) thereof to be 200-900. This medicine is effective as a medicine for preventing or suppressing the survival of cancer cells in a destination tissue.

## Description

### Cross-reference to the related applications

The present application is based upon and claims the benefit of priority from prior Japanese Patent Application No. 2013-162872, filed August 6, 2013, the entire contents of which are incorporated herein by reference.

### TECHNICAL FIELD

The present invention relates to a pharmaceutical use of an organic acid polymer, specifically to a 3-oxygermylpropionic acid polymer.

### BACKGROUND ART

Cancer treatment is based on early detection and early treatment. Examples of the treatment method include the following three kinds of therapies; surgical therapy resecting the site of lesions, and chemotherapy suppressing division or growth of cancer cells by administering a chemical substance, and radiation therapy suppressing division of cancer cells by radiating the cancer cells with X-ray or γ-ray. The present cancer treatment is carried out by the combination of these three therapies, according to the type of the cancer cells, and the site and stage of lesions.

A factor exerting a good influence on the record of cancer therapy is the inhibition of metastasis from the primary focus to new tissues. Metastasis is known to be caused by lymphatic metastasis, blood-borne metastasis, and dissemination. For example, blood-borne metastasis of cancer cells occurs through (1) invasion from the primary focus in neighborhood normal tissues; (2) invasion in blood vessels; (3) engraftment in the vascular endothelial cells of destination; (4) invasion in the tissues of destination; and (5) growth in the tissues of destination. If metastasis can be prevented or suppressed, the cancer therapy will be able to focus on only the primary cancer lesion intensively.

It has been gradually cleared that various molecular species are involved with each stage of metastasis of cancer cells. Examples of the cancer metastasis inhibitors for chemotherapy targeted at these molecular species under development include angiogenesis inhibitors, cell adhesion inhibitors, platelet aggregation inhibitors, signal transduction inhibitors, and extracellular matrix-degrading enzyme inhibitors, but they are not yet in actual use. Therapies in practical use such as chemotherapy are targeted at the division or growth of cancer cells.

Organic acid polymers, specifically a 3-oxygermylpropionic acid polymer, have been studied with respect to a number of pharmaceutical use. For example, uses for hypertensive cardiovascular diseases(see the following Patent Literature 1, the disclosures of which are hereby incorporated herein by reference), uses for hypertension (see the following Patent Literature 2, the disclosures of which are hereby incorporated herein by reference), uses for cataract (see the following Patent Literature 1, the disclosures of which are hereby incorporated herein by reference), an action of enhancing the production of interferons (see the following Patent Literature 3, the disclosures of which are hereby incorporated herein by reference), an action of inhibiting a Maillard reaction (see the following Patent Literature 4, the disclosures of which are hereby incorporated herein by reference), an action of enhancing the production of IL-10 (see the following Patent Literature 5, the disclosures of which are hereby incorporated herein by reference), uses for arteriosclerotic diseases (see the following Patent Literature 6, the disclosures of which are hereby incorporated herein by reference), an action of antagonizing MCP-1 (see the following Patent Literature 7, the disclosures of which are hereby incorporated herein by reference), uses for type II diabetic nephropathy (see the following Patent Literature 8, the disclosures of which are hereby incorporated herein by reference), uses for Crohn's disease (see the following Patent Literature 9, the disclosures of which are hereby incorporated herein by reference) and the like have been reported.

There are also reports on the anticancer effect of the 3-oxygermylpropionic acid polymer (see the following Patent Literatures 1, 2, and 10, the disclosures of which are hereby incorporated herein by reference), and an action of decreasing the CCL2 mRNA level in the adipose tissues (see the following Non-Patent Literature 1, the disclosures of which are hereby incorporated herein by reference). As mentioned above, many pharmaceutical uses have been reported on the 3-oxygermylpropionic acid polymer.

### Prior Art Technical Documents

### Patent Documents

Patent Document 1: Jpn. Pat. Appln. Laid-open Publication No. 55-167222
Patent Document 2: Jpn. Pat. Appln. Laid-open Publication No. 54-115324
Patent Document 3: Jpn. Pat. Appln. Laid-open Publication No. 2-134318
Patent Document 4: Jpn. Pat. Appln. Laid-open Publication No. 8-59485
Patent Document 5: Jpn. Pat. Appln. Laid-open Publication No. 11-49683
Patent Document 6: Jpn. Pat. Appln. Laid-open Publication No. 2000-229856
Patent Document 7: Jpn. Pat. Appln. Laid-open Publication No. 2000-136139
Patent Document 8: Jpn. Pat. Appln. Laid-open Publication No. 2003-81843
Patent Document 9: WO2011/093308
Patent Document 10: Jpn. Pat. Appln. Laid-open Publication No. 59-16825

### Non-Patent Documents

Non-Patent Document 1: J. Atheroscler. Thromb., 17(3), 219-228 (2010).

### SUMMARY OF INVENTION

### TECHNICAL PROBLEM

However, there is still no effective treatment method for the suppression of engraftment of cancer cells in the tissues of destination of the host cells. In addition, there is no report on the effect of the 3-oxygermylpropionic acid polymer on metastasis of cancer cells.

The present invention is intended to provide a new medicine for preventing or suppressing engraftments of cancer cells in the tissues of destination, thereby solving the prior art problems.

### SOLUTION TO PROBLEM

In view of the above-described problems, as a result of dedicated study by the inventors, the inventors found that Fbxw7, which is known as a cancer suppressor gene decomposing cancer gene proteins, regulates engraftment of cancer cells in the tissues of destination. More specifically, bone marrow-specific Fbxw7 knockout mice were prepared, and increase of CCL2 concentration in the blood, and metastasis of cancer cells were frequently found therein. The organic acid polymer represented by the below-described general formula (1), particularly the 3-oxygermylpropionic acid polymer was administered to the bone marrow-specific knockout mice; surprisingly, the increase of CCL2 concentration in the blood was suppressed, and further engraftment of cancer cells in the tissues of destination was successfully suppressed. The present invention has been accomplished based on these findings and successful examples. Accordingly, the scope of the present invention is as follows.

(1) A medicine for use in preventing or suppressing engraftment of cancer cells, comprising an organic acid polymer represented by the following general formula (I) as an active ingredient:

   [(O_{1/2})₃Ge-A-COOH]ₙ (I),

   wherein n represents an integer of 100 to 1000, and A represents a lower alkylene group.
(2) The medicine according to (1), wherein the n is an integer of 200 to 900.
(3) The medicine according to (1), wherein the A is a lower alkylene group having 1 to 3 carbon atoms.
(4) The medicine according to (1), wherein the organic acid polymer is a 3-oxygermylpropionic acid polymer.
(5) The medicine according to (1), wherein the organic acid polymer is a 3-oxygermylpropionic acid polymer showing the following powder X-ray diffraction spectrum:
   a large diffraction peak: in the vicinity of 6.5°; and
   relatively large diffraction peaks: in vicinity of 11.6°, 13.8°, 18.4°, 21.2°, and 22.4°.
(6) The medicine according to (1), wherein the organic acid polymer is a 3-oxygermylpropionic acid polymer showing the following infrared absorption spectrum:
   large absorption bands: in the vicinity of 800 cm⁻¹, 900 cm⁻¹, and 1700 cm⁻¹; and
   relatively large absorption bands: in the vicinity of 560 cm⁻¹, 705 cm⁻¹, 760 cm⁻¹, 780 cm⁻¹, 1250 cm⁻¹, 1350 cm⁻¹, and 1400 cm⁻¹, wherein the absorption band in the vicinity of 1400 cm⁻¹ is doublet.
(7) The medicine according to (1), wherein the organic acid polymer is a 3-oxygermylpropionic acid polymer showing the following chart in DSC:
   peak initiation point: in the vicinity of 237°C;
   peak top: in the vicinity of 256°C;
   peak end point: in the vicinity of 276°C; and
   amount of heat ΔH = approximately 59 mcal/mg.
(8) The medicine according to (1), wherein the organic acid polymer is a 3-oxygermylpropionic acid polymer represented by the following general formula (II) and having a weight average molecular weight of 9.29 × 10⁴ ± 5.72 × 10⁴ (average ± standard error):

   (C₃H₅GeO_{3.5})ₙ (II),

   wherein n represents a weight average degree of polymerization of 548 ± 337.
(9) The medicine according to (1), wherein the medicine is targeted at a cancer patient whose CCL2 concentration in the blood is higher than that in a normal subject.
(10) The medicine according to (9), wherein the cancer patient has dysfunction of the cancer suppressor gene Fbxw7 in the bone marrow.
(11) The medicine according to (1), wherein the organic acid polymer is for use in combination administration with one or more other chemotherapeutic agents selected from the group consisting of a molecularly targeted drug, an alkylating agent, an antimetabolite, a plant alkaloid, an anticarcinogenic antibiotic, a platinum, and a hormone.
(12) The medicine according to (11), wherein the other chemotherapeutic agents are one or more antimetabolites.
(13) The medicine according to (11), wherein the other chemotherapeutic agents are one or more antimetabolites selected from the group consisting of Gemcitabine, Tegafur-Uracil, and Fluorouracil.

### ADVANTAGEOUS EFFECT OF INVENTION

According to the present invention, a medicine which is highly safe for human body, and markedly prevents or suppresses engraftment of cancer cells in the destination tissues is provided.

### BRIEF DESCRIPTION OF DRAWING

FIG. 1 is a powder X-ray diffraction spectrum of 3-oxygermylpropionic acid polymer.
FIG. 2 is an infrared absorption (IR) spectrum of 3-oxygermylpropionic acid polymer.
FIG. 3 is a DSC chart of 3-oxygermylpropionic acid polymer.
FIG. 4 is a graph showing the CCL2 production suppressive effect of 3-oxygermylpropionic acid polymer; P = 0.011 {multiple comparison (Turkey-Kramer method)}.
FIG. 5 is a photograph showing engraftment of melanoma cells in the lungs at the time of anatomy.
FIG. 6 is a graph showing the rate of occupation by melanoma cells in the lung tissues; *** shows P < 0.001 {one-way analysis of variance / multiple comparison (Bonferroni method)}.

### MODE FOR CARRYING OUT THE INVENTION

The medicine of the present invention relates to a medicament for preventing or suppressing engraftment of cancer cells, the medicine comprising the organic acid polymer represented by the below-described general formula (I) as an active ingredient.

The organic acid polymer employed in the present invention is a known compound, and represented by the general formula (I):

[(O_{1/2})₃Ge-A-COOH]ₙ (I),

wherein n represents an integer of 100 to 1000, and A represents a lower alkylene group.

Here, the lower alkylene group represented by A is preferably a lower alkylene group having 1 to 3 carbon atoms. In particular, a 3-oxygermylpropionic acid polymer with ethylene group for A is preferable. In addition, the symbol n representing the degree of polymerization is preferably an integer of 200 to 900. In particular, the 3-oxygermylpropionic acid polymer having a degree of polymerization n of 200 to 900 is well known. Its steric structure is supposed to be an eight membered ring structure which is represented by the following formula: 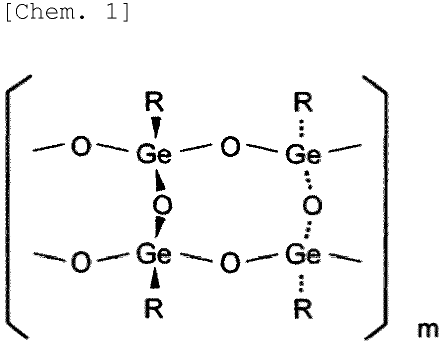 [wherein R represents -CH₂CH₂COOH, and m represents an integer of 137 ± 84 (average ± standard error 3σ) which is the weight average degree of polymerization converted from the weight average molecular weight of 3-oxygermylpropionic acid propyl ester polymer], the minimum unit being (O₁₁₂)₃GeCH₂CH₂COOH, and the compositional formula being C₆H₁₀Ge₂O₇.

The 3-oxygermylpropionic acid polymer can be prepared by the method described in, for example, Patent Document 8. In addition, the reference and others describe that the 3-oxygermylpropionic acid polymer shows the physical properties in the following Tables 1 and 2. Table 1 shows the result of molecular weight measurement by a light scattering method, and Table 2 shows the lattice constant obtained by powder X-ray diffraction analysis.

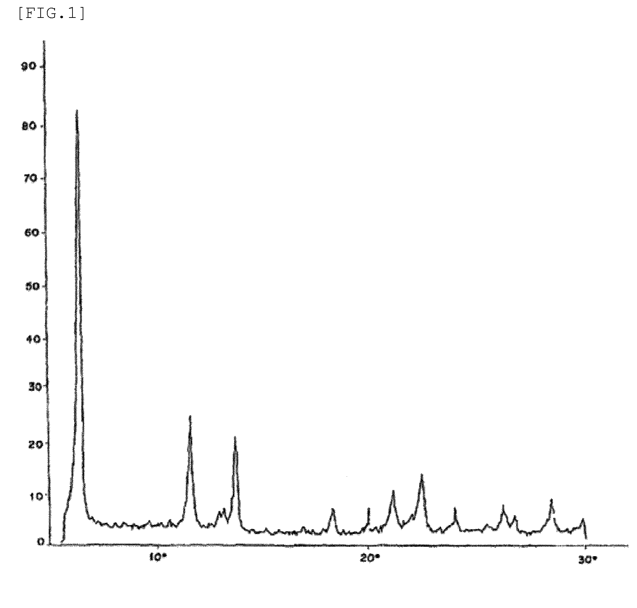

From Figs. 1 to 3 (cited from Patent Document 2), the 3-oxygermylpropionic acid polymer can be identified as a compound showing following physical properties. More specifically, it shows a characteristic powder X-ray diffraction spectrum with a large diffraction peak in the vicinity of 6.5°, and relatively large diffraction peaks in the vicinity of 11. 6°, 13.8°, 18.4°, 21.2°, and 22.4°, respectively; a characteristic infrared absorption (IR) spectrum with large absorption bands in the vicinity of 800 cm⁻¹, 900 cm⁻¹, and 1700 cm⁻¹, and relatively large absorption bands in the vicinity of 560 cm⁻¹, 705 cm⁻¹, 760 cm⁻¹, 780 cm⁻¹, 1250 cm⁻¹, 1350 cm⁻¹, and 1400 cm⁻¹ (wherein the absorption band in the vicinity of 1400 cm⁻¹ is doublet), respectively; and a characteristic DSC chart with a peak start point in the vicinity of 237°C, a peak top in the vicinity of 256°C, a peak end point of in the vicinity of 276°C, and an amount of heat ΔH of approximately 59 mcal/mg.

**[Table 1]**

| Weight average molecular weight of 3-oxygermylpropionic acid polymer | | |
|---|---|---|
| | 3-oxygermylpropionic acid polymer propyl ester | 3-oxygermylpropionic acid polymer (converted value) |
| Weight average molecular weight (Mw) | | |
| Average (x | 1.16 × 10⁵ | 9.29 × 10⁴ |
| Standard error (3σ) | ±0.71 × 10⁵ | ±5.72 × 10⁴ |
| Molecular formula | (C₆H₁₁GeO_{3.5})ₙ | (C₃H₅GeO_{3.5})ₙ |
| Weight average degree of polymerization (n) * | 548 ± 337 | 548 ± 337 |
| * is a value when the minimum unit of 3-oxygermylpropionic acid polymer is (O_{1/2}) ₃GeCH₂CH₂COOH. | | |

**[Table 2]**

| | |
|---|---|
| Lattice constant of 3-oxygermylpropionic acid polymer | |
| Chemical formula*¹ | (C₃H₅GeO_{3.5})ₙ |
| Formula weight*¹ | 169.66 |
| Crystal class | monoclinic |
| Space group | --- |
| Unit cell parameters | |
| a (Å) | 13.35*¹ |
| b (Å) | 5.03*¹ |
| c (Å) | 7.55*¹ |
| β (deg.) | 94.3*¹ |
| vol (Å³) | 505.4*² |
| z | 4*³ |
| density (gem⁻³) | 2.23*⁴ |
| *1 The repeating unit of 3-oxygermylpropionic acid polymer is represented as (O_{1/2})₃GeCH₂CH₂COOH | |
| *2 Calculated from the lattice constant | |
| *3 Calculated from the lattice constant and measured density | |
| *4 Measured by a flotation method | |

More specifically, a preferred embodiment of 3-oxygermylpropionic acid polymer is the 3-oxygermylpropionic acid polymer represented by the following general formula (II) :

(C₃H₅GeO_{3.5})ₙ (II)

(wherein n represents a weight average degree of polymerization of 548 ± 337), the weight average molecular weight being 9.29 × 10⁴ ± 5.72 × 10⁴ (average ± standard error).

Fbxw7, which is known as a ubiquitinating enzyme, mediates degradation of cancer-gene proteins, thereby playing an important role as a cancer suppressor gene suppressing cancer in the living body. The inventors have found that Fbxw7 has a new function; it acts on the cancer cells in the host tissues, specifically the bone marrow-derived mesenchymal stem cells, thereby controlling engraftment of cancer cells to the host tissues. The "engraftment" in the tissues of destination of cancer cells means the stage where the cancer cells liberated from the primary site adhere to, for example, the endothelial cells in the lymphatic or blood vessels, or the serosa in the body cavity, and infiltrate and start to growth in the new tissues.

The Fbxw7 knockout mice are embryonic lethal, so that tissue-specific Fbxw7 knockout model mice were prepared. Enhancement of engraftment of externally introduced cancer cells to the homing destination of the host tissues was not found even though the Fbxw7 derived from other than bone marrow, such as T cells, B cells, and myeloid cells was knocked out. On the other hand, enhancement of engraftment of externally introduced cancer cells to the homing destination of the host tissues was found only when the Fbxw7 was knocked out specifically in the bone marrow. This result shows that the bone marrow-derived Fbxw7 inhibits engraftment of cancer cells in the host tissue.

CCL2 is known to be involved with cancer metastasis (see R. D. Loberg, et. al., Neoplasia 9(7), 556-562 (2007), the disclosures of which are hereby incorporated herein by reference). In the bone marrow-specific Fbxw7 knockout model mice, the CCL2 concentration in the blood increased. Therefore, in this model mice, engraftment of cancer cells introduced from outside may be facilitated owing to the increase in the CCL2 concentration in the blood. Accordingly, the use of the above-described model mice allows evaluation of various drugs for the effect of suppressing engraftment of cancer cells in blood to the homing destination of the host tissues.

The compounds showing engraftment suppressive effect were screened using the above-described model mice; the 3-oxygermylpropionic acid polymer was found to have effect of suppressing engraftment of melanoma cells in the lungs. Furthermore, as mentioned in the below-described examples, the administration of the 3-oxygermylpropionic acid polymer to the above-described model mice decreased the CCL2 concentration in the blood to the control group level. The 3-oxygermylpropionic acid polymer decreases the CCL2 concentration in the host blood of the CCL2-CCR2 system related to cancer cell metastasis, thereby possibly suppressing engraftment of the cancer cells in blood to the homing destination of the host tissues.

The drug decreasing the CCL2 secretion of the host is effective in cancer cell metastasis by CCL2 supersecretion, irrespective of the type of cancer. More specifically, the cancer patient whose CCL2 concentration in the blood is higher than that in a normal subject is a preferred target of the medicine of the present invention. The cancer cells to be treated are not particularly limited, and all metastatic cancer cells are the objects. Examples include stomach cancer, penis cancer, liver cancer, thyroid cancer, uterus cancer, esophagus cancer, kidney cancer, renal pelvis and ureter cancer, pancreas cancer, testis cancer, prostate gland cancer, large intestine cancer, biliary cancer, head and neck cancer, breast cancer, cerebral tumor, lung cancer, skin cancer, adrenal glands cancer, bladder cancer, and ovarian cancer. In particular, high effect is expected for pancreas cancer, breast cancer, skin cancer, and prostate gland cancer. Examples of the cancer patients to whom high effect is expected include the cancer patients having dysfunction of the cancer suppressor gene Fbxw7 in the bone marrow.

The 3-oxygermylpropionic acid polymer used in the present invention is a medicine of novel type which suppresses engraftment of cancer cells, and thus may be administered in combination with, for example, prior art surgical treatment, radiation therapy, and/or chemotherapy. Examples of the other anticancer agent which may be administered in combination with the medicine of the present invention are listed below.

Examples of the molecularly targeted drugs usable as other anticancer agents include Ibritumomab, Tiuxetan, Imatinib, Everolimus, Erlotinib, Gefitinib, Gemtuzumab, Ozogamicin, Sunitinib, Cetuximab, Sorafenib, Dasatinib, Tamibarotene, Trastuzumab, Tretinoin, Panitumumab, Bevacizumab, Bortezomib, Lapatinib, Rituximab, and the like.

Examples of the alkylating agents usable as other anticancer agents include Ifosfamide, Cyclophosphamide, Dacarbazine, Temozolomide, Nimustine, Busulfan, Procarbazine, Melphalan, Ranimustine, and the like.

Examples of the antimetabolites usable as other anticancer agents include Enocitabine, Capecitabine, Carmofur, Cladribine, Gemcitabine, Cytarabine, Cytarabine Ocfosfate, Tegafur, Tegafur-Uracil, Tegafur-Gimeracil-Oteracil Potassium, Doxifluridine, Nelarabine, Hydroxylcarbamide, Fluorouracil, Fludarabine, Pemetrexed, Pentostatin, Mercaptopurine, Methotrexate, and the like. Among these antimetabolites, combination with Gemcitabine, Tegafur-Uracil, or Fluorouracil can achieve high cancer therapeutic effect.

Examples of the plant alkaloids usable as other anticancer agents include Irinotecan, Etoposide, Eribulin, Sobuzoxane, Docetaxel, Nogitecan, Paclitaxel, Paclitaxel Injection, Vinorelbine, Vincristine, Vindesine, Vinblastine, and the like.

Examples of the anticarcinogenic antibiotics usable as other anticancer agents include Actinomycin D, Aclarbicin, Amrubicin, Idarubicin, Epirubicin, Zinostatin Stimalamer, Daunorubicin, Doxorubicin, Pirarubicin, Bleomycin, Peplomycin, Mitomycin C, Mitoxantrone, Liposomal Doxorubicin, and the like.

Examples of the platinums usable as other anticancer agents include Oxaliplatin, Carboplatin, Cisplatin, Nedaplatin, and the like.

Examples of the hormones usable as other anticancer agents include Anastrozole, Exemestane, Etramustine, Ethinylestradiol, Chlormadinone, Goserelin, Tamoxifen, Dexamethasone, Tremifene, Bicalutamide, Flutamide, Prednisolone, Fosfestrol, Mitotane, Methyltestosterone, Medroxyprogesterone, Mepitiostane, Leuprorelin, Letrozole, and the like.

The surgical treatment, radiation therapy, chemotherapy and the like used in combination with the medicine of the present invention may be carried out before or after, or concurrently with the administration of the medicine of the present invention. For example, when other anticancer agent is used, it is administered before or after, or concurrently with the administration of the medicine of the present invention. When the medicine of the present invention and other anticancer agent are concurrently administered in combination, they can be used in separate forms, or in an integral form such as a combination drug.

The 3-oxygermylpropionic acid polymer was evaluated using the bone marrow-specific Fbxw7 knockout mice, and found to act on the host cells to suppress engraftment of cancer cells. The 3-oxygermylpropionic acid polymer is a novel engraftment suppressor which is independent of cancer cells and compatible with existing chemotherapeutic agents. Accordingly, when the polymer is administered to a cancer patient who has already caused metastasis, engraftment of cancer cells in the tissues of destination is suppressed, whereby further metastasis of cancer cells can be suppressed. In addition, when the polymer is administered to a cancer patient who has not caused metastasis, engraftment of cancer cells to the homing destination of the host tissues is prevented, whereby metastasis of cancer cells can be prevented.

When the medicine of the present invention is actually administered to a human, it is a preferably used as a composition containing 0.005 to 50 parts by mass of an excipient with respect to 0.005 to 5 parts by mass of 3-oxygermylpropionic acid polymer. Examples of the excipient include, but not limited to, saccharides such as lactose, sucrose, or dextrans; cellulose polymer substances such as hydroxypropyl cellulose; and natural polymer substances such as albumin. In addition, the compound is usually used in the form of an oral formulation, and may be used in the form of, for example, a suppository, a nasal cavity formulation, or injection formulation. When the compound is administered to a human, the dose depends on the dosage form , age of the patient, and the like, and, for example, from 1 mg to 1500 mg a day. When orally administered to an adult having a body weight of 60 kg, the dose is preferably 10 mg to 120 mg a day. In addition, formulation of the compound may be carried out in accordance with the description of the formulation example in, for example, Patent Document 8.

The present invention is further explained in detail by the following examples, but these examples will not limit the present invention, and may be modified without departing from the scope of the present invention.

### Examples

### Preparation: preparation of 3-oxygermylpropionic acid polymer

Germanium dioxide and 50% (w/v) hypophosphorous acid (1.1-fold mol of germanium dioxide, hereinafter the same) were reacted in the presence of concentrated hydrochloric acid (5.0-fold mol) at 60 to 80°C for 4 hours. To the reactant thus obtained, concentrated hydrochloric acid (5.0-fold mol) was added, and acrylic acid (1.1-fold mol) was added dropwise thereto at 40°C or lower. The crystal precipitated by this operation was collected by filtration, and washed with concentrated hydrochloric acid to obtain 3-trichlorogermylpropionic acid (yield: 98%). The 3-trichlorogermylpropionic acid thus obtained was dissolved in acetone (17-fold mol), and then filtered. To the filtrate thus obtained, water (70-fold mol) was added dropwise at 0°C under stirring. The solution thus obtained was stirred for 6 hours, and allowed to stand for 16 hours. The crystal precipitated by this operation was collected by filtration, and washed with acetone to obtain 3-oxygermylpropionic acid polymer (yield: 92%).

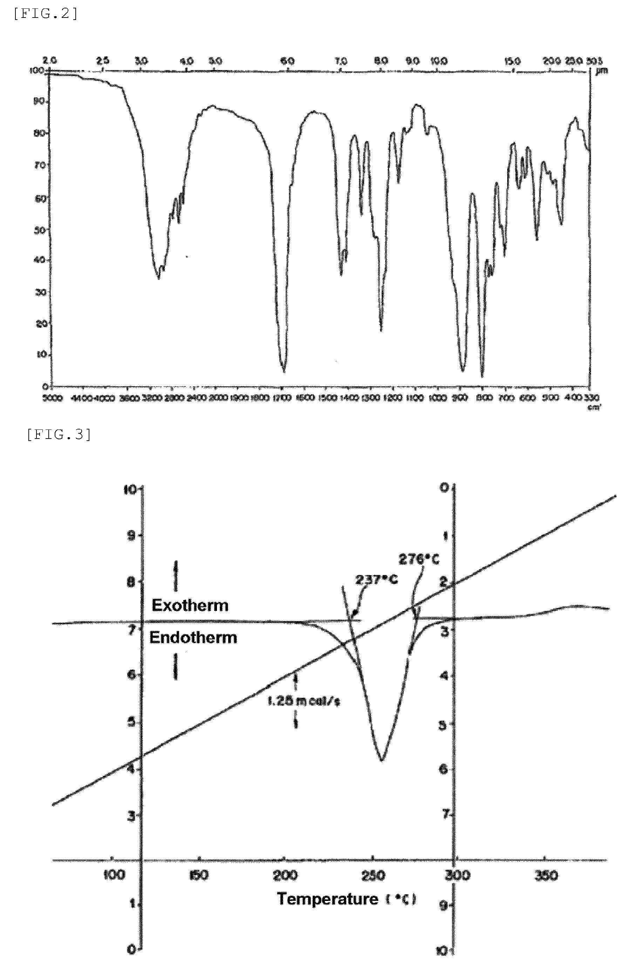

The 3-oxygermylpropionic acid polymer thus obtained was induced to a propyl ester. The molecular weight of the propyl ester was measured by light scattering method, and the lattice constant by powder X-ray diffraction was measured. The results were as shown in the above-described Tables 1 and 2, and Fig. 1, respectively. In addition, the infrared absorption (IR) spectrum of the propyl ester was as shown in Fig. 2, and the DSC chart was as shown in Fig. 3. The measurement conditions of the DSC chart were as shown in the following Table 3.

**[Table 3]**

| | |
|---|---|
| Sample weight | 9.87 mg |
| Control | Alumina |
| DSC range | 250 µV |
| Temperature range | 20 mV |
| Chart speed | 1 cm/minute |
| Temperature rising velocity | 10°C/minute |
| N2 | 20 ml/minute |
| Open container made of Al (crimp) | |
| Amount of heat ΔH | 59.4 mcal/mg |

### Test Example 1: preparation of bone marrow-specific Fbxw7 knockout mice

As the bone marrow-specific Fbxw7 knockout group, an 8-week-old Mx1-Cre/Fbxw7^{F/F} mouse (see Matsuoka, S. et al., Genes Dev. 22, 986-991 (2008), the disclosures of which are hereby incorporated herein by reference) (n = 14) prepared by mating an Fbxw7^{F/F} mouse (Onoyama, I. et al., J. Exp. Med. 204, 2875-2888 (2007), the disclosures of which are hereby incorporated herein by reference) with an Mx1-Cre transgenic mouse (see Kuhn, R., Schwenk, F., Aguet, M. & Rajewsky, K. Science 269, 1427-1429 (1995), the disclosures of which are hereby incorporated herein by reference) was used. To the bone marrow-specific Fbxw7 knockout group, polyinosine-polycytidine acid (pIpC; Cal Bio-Chem Co., Ltd.) was administered at a rate of 20 µg per gram of body weight, six times at a frequency of once every other day, thereby inducing defect of Fbxw7 Flox gene locus. The defect of the Fbxw7 Flox gene locus was confirmed by PCR analysis of genome DNA. The same amount of pIpC was administered in the same manner as above to the control group (8-week-old Fbxw7 ^{F/F} mice; n = 5) which has not been subjected to the induction of defect of the Fbxw7 Flox gene locus. The feed was a mouse standard feed NMF (Oriental Yeast Co., Ltd.). As described above, experimental model mice were prepared.

### Test Example 2: CCL2 silencing

[Test method] First blood collection from the experimental model mice prepared in the above-described Test Example 1 was carried out on day 4 after completion of the administration of pIpC, and recorded as 0 week. To the specimen administration group, the feed was changed immediately after completion of blood collection to the 3-oxygermylpropionic acid polymer-containing feed, which had been prepared by mixing a mouse standard feed NMF (Oriental Yeast Co., Ltd.) with 0.015% (w/w) of the 3-oxygermylpropionic acid polymer, and its administration was started. A mouse standard feed NMF (Oriental Yeast Co., Ltd.) containing no 3-oxygermylpropionic acid polymer was administered to the specimen non-administration group. Blood collection was carried out on weeks 2 and 3 of administration, and the CCL2 concentration in the blood was measured by ELISA. The measurement used Mouse CCL2 (MCP-1) ELISA Ready-SET-Go! (registered trademark, eBioscience, Inc.), and the experiment was carried out in accordance with the protocol attached to the kit.

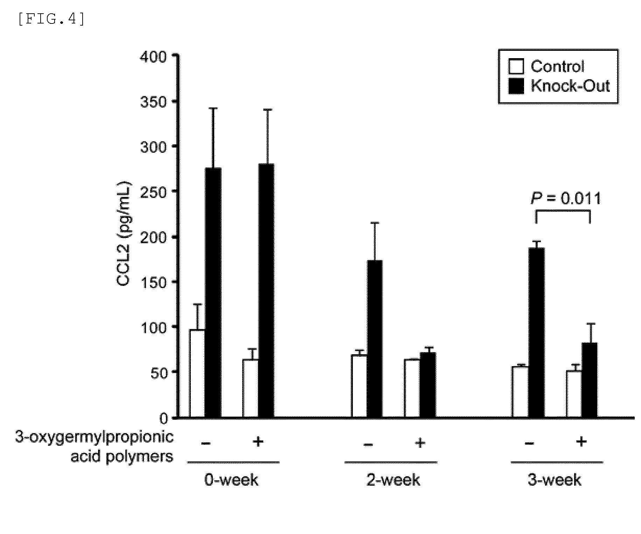

[Result] It was found that the CCL2 concentration in the blood of the specimen administration group on week 0 was enhanced in the bone marrow-specific Fbxw7 knockout group in comparison with the control group (Fig. 4). This result shows that the bone marrow-specific defect of Fbxw7 influences the production of CCL2 on the host side.

For the bone marrow-specific Fbxw7 knockout group in the specimen administration group to which the 3-oxygermylpropionic acid polymer was administered, the CCL2 amount in the blood significantly decreased from week 2 in comparison with the bone marrow-specific Fbxw7 knockout group in the specimen non-administration group, and became almost the same level as that in the control group (Fig. 4). This result shows that the 3-oxygermylpropionic acid polymer suppresses the production of CCL2 in the host side. The CCL2 concentration in the blood was rather higher on week 0, which is likely due to the influence of the type I interferon induction by the administration of pIpC on the CCL2 amount on week 0.

### Test Example 3: Analysis of cancer engraftment

### [Test Method]

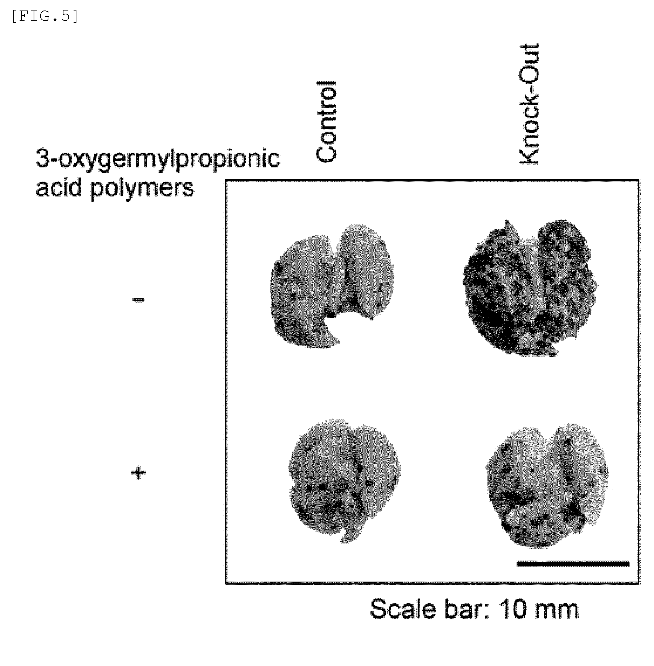

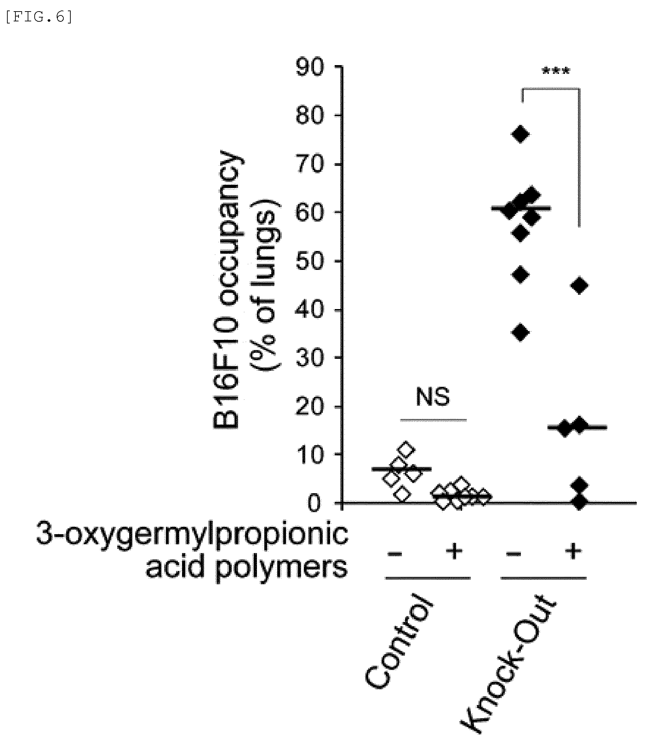

To the experimental model mice prepared in Test Example 1, the administration of a feed containing the 3-oxygermylpropionic acid polymer, which had been prepared by mixing 0.005% (w/w) of the 3-oxygermylpropionic acid polymer with a mouse standard feed NMF (Oriental Yeast Co., Ltd.) to the specimen administration group was started on day 2 after the completion of administration of pIpC. 2.0 × 10⁵ mouse melanoma cells B16F10 were transplanted through the tail vein on day 1 after the initiation of administration of the 3-oxygermylpropionic acid polymer-containing feed. Anatomy was carried out on day 14 after transplantation, the lungs were removed, and the peripheral blood was collected. The average dose of the 3-oxygermylpropionic acid polymer-containing feed during the administration period was about 5 mg/kg a day based on the body weight and food consumption. A mouse standard feed NMF (Oriental Yeast Co., Ltd.) containing no 3-oxygermylpropionic acid polymer was administered to the specimen non-administration group. The lung occupancy of melanoma cells was calculated as follows: the removed lungs were fixed by Bouin's solution, and then the occupancy of melanoma cells on the lung surface was calculated by image processing using Image J (Figs. 5 and 6).

[Result] In the control group, there was no significant difference in the number of colonies of melanoma cells metastasized to the lungs, irrespective of administration or non-administration of the 3-oxygermylpropionic acid polymer (Fig. 5). This result shows that, in this test system, the 3-oxygermylpropionic acid polymer will not directly act on cancer cells to develop anticancer activity.

In the specimen non-administration group, the number of colonies of melanoma cells engrafted in the lungs was about seven times higher in the bone marrow-specific Fbxw7 knockout group, in comparison with the control group. On the other hand, in the bone marrow-specific Fbxw7 knockout group, in the specimen administration group, engraftment of melanoma cells in the lungs was markedly suppressed in comparison with the specimen non-administration group (Figs. 5 and 6).

These results show that the bone marrow-specific Fbxw7 has inhibitory effect on engraftment of cancer cells, and that the 3-oxygermylpropionic acid polymer has suppressive effect on engraftment of cancer cells in tissues.

## Claims

1. A medicine for use in preventing or suppressing engraftment of cancer cells, comprising an organic acid polymer represented by the following general formula (I) as an active ingredient:
[(O_{1/2})₃Ge-A-COOH]ₙ (I),
wherein n represents an integer of 100 to 1000, and A represents a lower alkylene group.

2. The medicine according to claim 1, wherein the n is an integer of 200 to 900.

3. The medicine according to claim 1, wherein the A is a lower alkylene group having 1 to 3 carbons.

4. The medicine according to claim 1, wherein the organic acid polymer is a 3-oxygermylpropionic acid polymer.

5. The medicine according to claim 1, wherein the organic acid polymer is a 3-oxygermylpropionic acid polymer showing the following powder X-ray diffraction spectrum:
a large diffraction peak: in the vicinity of 6.5°; and
relatively large diffraction peaks: in the vicinity of 11.6°, 13.8°, 18.4°, 21.2°, and 22.4°.

6. The medicine according to claim 1, wherein the organic acid polymer is a 3-oxygermylpropionic acid polymer showing the following infrared absorption spectrum:
large absorption bands: in the vicinity of 800 cm⁻¹, 900 cm⁻¹, and 1700 cm⁻¹; and
relatively large absorption bands: in the vicinity of 560 cm⁻¹, 705 cm⁻¹, 760 cm⁻¹, 780 cm⁻¹, 1250 cm⁻¹, 1350 cm⁻¹, and 1400 cm⁻¹, wherein the absorption band in the vicinity of 1400 cm⁻¹ is doublet.

7. The medicine according to claim 1, wherein the organic acid polymer is a 3-oxygermylpropionic acid polymer showing the following chart in DSC:
peak initiation point: in the vicinity of 237°C;
peak top: in the vicinity of 256°C;
peak end point: in the vicinity of 276°C; and
amount of heat ΔH = approximately 59 mcal/mg.

8. The medicine according to claim 1, wherein the organic acid polymer is a 3-oxygermylpropionic acid polymer represented by the following general formula (II) and having a weight average molecular weight of 9.29 × 10⁴ ± 5.72 × 10⁴ (average ± standard error):
(C₃H₅GeO_{3.5})ₙ (II),
wherein n represents a weight average degree of polymerization of 548 ± 337.

9. The medicine according to claim 1, wherein the medicine is targeted at a cancer patient whose CCL2 concentration in the blood is higher than that in a normal subject.

10. The medicine according to claim 9, wherein the cancer patient has dysfunction of the cancer suppressor gene Fbxw7 in the bone marrow.

11. The medicine according to claim 1, wherein the organic acid polymer is for use in combination administration with one or more other chemotherapeutic agents selected from the group consisting of a molecularly targeted drug, an alkylating agent, an antimetabolite, a plant alkaloid, an anticarcinogenic antibiotic, a platinum, and a hormone.

12. The medicine according to claim 11, wherein the other chemotherapeutic agents are one or more antimetabolites.

13. The medicine according to claim 11, wherein the other chemotherapeutic agents are one or more antimetabolites selected from the group consisting of Gemcitabine, Tegafur-Uracil, and Fluorouracil.
